# EUROPEAN PATENT SPECIFICATION

(11) **EP 2 551 343 B1**
(45) Date of publication and mention of the grant of the patent: **02.05.2018**
(21) Application number: 11759460.6
(22) Date of filing: 23.03.2011
(51) Int. Cl.: C12N 5/10, C12Q 1/02, C12Q 1/66, C12N 15/12, C12Q 1/68, G01N 33/50, G01N 33/58

(54) **METHOD FOR MONITORING STATE OF DIFFERENTIATION IN A STEM CELL**
VERFAHREN ZUR ÜBERWACHUNG DES DIFFERENZIERUNGSSTATUS EINER STAMMZELLE
PROCÉDÉ POUR SURVEILLER L'ÉTAT DE DIFFÉRENCIATION DANS UNE CELLULE SOUCHE

(30) Priority: 28.02.2011 JP 2011042870; 23.03.2010 JP 2010066896
(43) Date of publication of application: 30.01.2013
(73) Proprietor: Olympus Corporation, Tokyo 192-8507 (JP)
(72) Inventor: OHASHI, Yoko, Tokyo 192-8507 (JP)
(74) Representative: Gunzelmann, Rainer
(86) International application number: PCT/JP2011/057034
(87) International publication number: WO 2011/118655

(56) References cited:
- WO-A1-03/018780
- WO-A1-2006/106882
- WO-A1-2011/029798
- JP-A- 2002 508 670
- US-A1- 2004 096 432
- US-A1- 2005 287 127
- AUBERT J ET AL: "Functional gene screening in embryonic stem cells implicates Wnt antagonism in neural differentiation", NATURE BIOTECHNOLOGY, NATURE PUBLISHING GROUP, NEW YORK, NY, US, vol. 20, no. 12, 1 January 2002 (2002-01-01), pages 1240-1245, XP002904121, ISSN: 1087-0156, DOI: 10.1038/NBT763
- ASAI S ET AL.: 'Single-cell imaging of c-fos expression in rat primary hippocampal cells using a luminescence microscope.' NEUROSCI. LETT. vol. 434, 2008, pages 289 - 292
- WELSH D K ET AL.: 'Bioluminescence imaging of individual fibroblasts reveals persistent, independently phased circadian rhythms of clock gene expression.' CURR. BIOL. vol. 14, 2004, pages 2289 - 2295
- CARR A-J F ET AL.: 'Imaging of single light-responsive clock cells reveals fluctuating free-running periods.' NAT. CELL BIOL. vol. 7, 2005, pages 319 - 321
- NOGUCHI T ET AL.: 'Simultaneous monitoring of independent gene expression patterns in two types of cocultured fibroblasts with different color-emitting luciferases.' BMC BIOTECHNOL., [Online] vol. 8, no. 40, 2008, Retrieved from the Internet: <URL:http://dx.doi.org/10.1186/1472-6750-8- 40>
- Timm Schroeder: "Tracking Hematopoiesis at the Single Cell Leve", Annals of the New York Academy of Science, 9 January 2006 (2006-01-09), pages 201-209, XP055131231, Retrieved from the Internet: URL:http://onlinelibrary.wiley.com/doi/10. 1196/annals.1349.025/abstract [retrieved on 2014-07-23]
- STADTFELD MATTHIAS ET AL: "Fluorescent protein-cell labeling and its application in time-lapse analysis of hematopoietic differentiation.", METHODS IN MOLECULAR MEDICINE 2005, vol. 105, 2005, pages 395-412, ISSN: 1543-1894

### SEQUENCE LISTING

<110> Olympus Corporation
<120> A method for monitering the state of differentiation of a stem cell
<130> 11P00703
<150> JP 2010-066896
   <151> 2010-03-23
<150> JP 2011-042870
   <151> 2011-02-28
<160> 4
<170> PatentIn version 3.3
<210> 1
   <211> 25
   <212> DNA
   <213> Artificial
<220>
   <223> Forward primer for amplifying promoter region of Nanog gene
<400> 1
   ctactcgaga tcgccagggt ctgga 25
<210> 2
   <211> 28
   <212> DNA
   <213> Artificial
<220>
   <223> Reverse primer for amplifying promoter region of Nanog gene
<400> 2
   ctactcgagc gcagccttcc cacagaaa 28
<210> 3
   <211> 27
   <212> DNA
   <213> Artificial
<220>
   <223> Forward primer for amplifying promoter region of Nestin gene
<400> 3
   gagaacgcgt gggctgtgtg ttgcact 27
<210> 4
   <211> 32
   <212> DNA
   <213> Artificial
<220>
   <223> Reverse primer for amplifying promoter region of Nestin gene
<400> 4
   gagactcgag gtggagcact agagaaggga gt 32

## Description

### Technical Field

The present invention relates to a method for monitoring the differentiation state of a stem cell , as defined in the claims.

### ackground Art

In the regenerative medicine field, cell transplantation using a stem cell and intractable disease treatment by organ regeneration are expected. While clinical application of a stem cell to heart disease and the like is precedent, there are many unclear points on how the transplanted stem cell actually works. In order to perform safe transplantation, it is essential to elucidate the mechanism of differentiation induction of a stem cell (induction to a cell having a specific function) and identify the factors controlling the intracellular dynamics and function.

So far, it has been found that a stem cell expresses some transcription factors highly specific to an undifferentiated cell at the molecular level. These include Oct-4, Sox, Nanog, and leukemia inhibitory factor receptor (LIF-R). Oct-4 is expressed in a pre-gastrulation embryo, an early cleavage stage embryo, a cell of the inner cell mass of the blastocyst, and an embryonal carcinoma (EC) cell. In an adult animal, Oct-4 is found only in a germ cell.

In a stem cell experiment involving an ES cell, iPS cell or the like, pluripotency is maintained by co-culture with a feeder cell or addition of LIF, but the cell may lose pluripotency because of the environment or cell conditions and easily differentiate. In an attempt to understand the differentiation induction process, it is important to exactly identify the undifferentiation state (state of having pluripotency) or the differentiation state, but it is difficult to determine the differentiation state only by the morphological information of an ES cell.

As an index for determining pluripotency, alkaline phosphatase staining and immunostaining using a specific antibody to a differentiation marker have been performed. Both methods involve immobilization step of the cell, thus the state of the cell cannot be continuously investigated.

Stem cell research is expected to be used in the regenerative medicine field such as transplantation therapy, and thus it is desirable to observe the time-course changes of the state of the stem cell in the living state. By observing the cell in the living state, it is also possible to isolate the stem cell suited for the purpose of the experiment or the differentiation-induced cell. Even from the viewpoint of basic research, there are many unclear points as to the mechanism of maintaining the undifferentiated state of the stem cell or as to change of the marker gene expression level during differentiation. Therefore, it is necessary to study with a living stem cell for elucidating the mechanism of differentiation induction.

For the purpose of investigating the induced differentiation state of the cell in the living state, a reporter assay is carried out using GFP as a reporter to observe the expression level of a differentiation marker (Patent Document 1, JP 2008-118991). WO 2011/029798 discloses in vitro monitoring of differentiation of mouse embryonic stem cells expressing luciferase under control of an Oct-4 promoter by means of a CCD camera system, but not of each stem cell in the colony.

US 2004/096432 A1 discloses monitoring of stem cells expressing a reporter construct of EGFP under the control of a cardiac-specific promoter.

### Summary of Invention

### Problem to be Solved by the Invention

The present inventors focused on that, when a method of Patent Document 1 (Jpn. Pat. Appln. KOKAI Publication No. 2008-118991) is utilized in monitoring the differentiation state of a stem cell over a given period, there are problems as described below.

Specifically, when a fluorescent protein gene such as GFP is used as a reporter as described in Patent Document 1, considerable noise occurs due to excitation light irradiation, thus quantification of the gene expression level is sometimes difficult. Therefore, fluorescence observation using GFP as an index is often used as a method for identifying the presence or absence of gene expression. In addition, when GFP is used as a reporter, an excitation light is irradiated during observation, thus the effect of damaging a cell is also predicted in the case of a long term observation for several days to several weeks.

Furthermore, in Patent Document 1, a flow cytometer is used for isolating a stem cell. In the case of performing flow cytometry detection after unifying a stem cell (or colony derived from a stem cell), it is also possible that an intended stem cell cannot be selected, because gene expression level may change at the stage of the cell cycle in the stem cell.

As described above, since an excitation light is irradiated in the method of investigating the differentiation state of a cell by a reporter assay using GFP as an index, there are problems in quantitative property and a long term observation.

On the other hand, as a method for detecting the intracellular gene expression level without irradiating an excitation light, a detection is performed using a luminescent protein-coding gene such as a luciferase gene as a reporter. Since luciferase emits light by an enzyme-substrate reaction with luciferin, introduction of excitation light is unnecessary, and a long term observation is possible. However, detection of gene expression change using luciferase is generally carried out by means of photon counting detection with a luminometer, as described in Patent Literature 2 (Jpn. PCT National Publication No. 2009-523025). In this detection, the gene expression change is detected as the integration of cell population in the entire dish, and therefore the gene expression change of each individual stem cell cannot be detected, when a stem cell (or a colony derived from a stem cell) which is considered as a heterogenous cell population *in vivo* is used as an object of detection. In addition, the colony derived from a stem cell is a three-dimensional aggregate of various cells, thus it is difficult to understand the gene expression level of cells in the state of the three-dimensional aggregate. Moreover, the colony derived from a stem cell is different in characteristics by each stem cell and each colony. Therefore, in the conventional method for observing entire cell population in a dish, there is a problem in that exact analysis by each stem cell and colony is difficult.

Focusing on the above problems, an object of the present invention is to provide a method for monitoring the differentiation state of stem cell in each cell or each colony without damage to the cell.

### Means for Solving the Problem

The present inventors introduced a fusion gene of a promoter region of a differentiation state detection marker gene and a luminescent protein-coding gene (luciferase gene) into a stem cell and captured images of luminescence emitted by expression of the luminescent protein-coding gene (luciferase gene) in the stem cell over a given period, thereby achieving the above object and completing the present invention. More specifically, the present invention provides the following means.
[1] A method for monitoring the differentiation state of a stem cell, comprising:
   a step (A-1) of culturing a stem cell into which a fusion gene of a promoter region of a differentiation state detection marker gene and a luciferase-coding gene is transfected;
   a step (A-2) of culturing the stem cell under a differentiation-inducing condition after the step (A-1); and
   a step (A-3) of capturing images of luminescence emitted by expression of the luciferase-coding gene in the stem cell, over at least a given period of the steps (A-1) to (A-2), as further defined in the claims.
[2] A method for monitoring the differentiation state of a stem cell, comprising:
   a step (B-1) of culturing a stem cell into which a fusion gene of a promoter region of a differentiation state detection marker gene and a luciferase-coding gene is transfected;
   a step (B-2) of further subculturing the stem cell;
   a step (B-3) of culturing the stem cell under a differentiation-inducing condition after the step (B-2); and
   a step (B-4) of capturing images of luminescence emitted by expression of the luciferase-coding gene in the stem cell, over at least a given period of the steps (B-1) to (B-3), as further defined in the claims.
[3] A method for monitoring the differentiation state of a stem cell, comprising:
   a step (C-1) of culturing a stem cell into which a fusion gene of a promoter region of a differentiation state detection marker gene and a luciferase-coding gene is transfected;
   a step (C-2) of further subculturing the stem cell; and
   a step (C-3) of capturing images of luminescence emitted by expression of the luciferase-coding gene in the stem cell, over at least a given period of the steps (C-1) to (C-2), as further defined in the claims.
[4] The method according to any one of the above [1] to [3], wherein the image capturing step is a step of capturing multiple luminescence images continuously by a luminescence imaging system.
[5] The method according to the above [4], further comprising a step of measuring the luminescence intensity as an index of the differentiation state in a specific cellular region of the luminescence images obtained in the image capturing step, and analyzing a temporal change of the luminescence intensity.
[6] The method according to any one of the above [1] to [5], further comprising a step of performing bright field observation and/or fluorescence observation for obtaining the information of the contour and/or site of the stem cell or the colony to which the stem cell belongs, and obtaining a bright field image and/or fluorescence image in the same region as the luminescence images.
[7] The method according to any one of the above [1] to [6],
   wherein the stem cell is a stem cell into which plural types of fusion genes are transfected,
   each fusion gene contains a promoter region of a different type of a differentiation state detection marker gene, and
   promoter region of the differentiation state detection marker gene is fused with a gene encoding each luciferase having a different spectral characteristic of luminescence, so as to be detected distinguishably from a promoter region of other differentiation state detection marker gene.
[8] The method according to the above [7], wherein the stem cell is a stem cell into which two types of fusion genes are transfected,
   one of the fusion genes is a fusion gene of a promoter region of an undifferentiation marker gene and a first type of a luciferase-coding gene, and
   the other is a fusion gene of a promoter region of a differentiation marker gene which is specifically expressed in a specific differentiation process and a second type of a luciferase-coding gene whose expression is detected distinguishably from the expression of the first type of the luminescent protein-coding gene.
[9] A method for identifying the differentiation state of a stem cell, comprising a step of identifying the differentiation state of a stem cell, based on the luminescence image and/or luminescence intensity data and/or bright field image obtained by the method according to any one of the above [1] to [8].
[10] A method for collecting a stem cell showing a desired differentiation state, comprising a step of identifying the differentiation state of a stem cell, based on the luminescence image and/or luminescence intensity data and/or bright field image obtained by the method according to the above [6]; and a step of picking up a stem cell showing a desired differentiation state from a plurality of stem cells.
[11] The method according to any one of the [1] to [10], wherein the luminescence image indicates the gene expression level of an individual stem cell in a colony.
[12] The method according to the above [11], wherein the colony forms an embryoid body.

### Effects of Invention

According to the method of the present invention, a fusion gene of a promoter region of a differentiation state detection marker gene and a luminescent protein-coding gene (luciferase gene) is transfected into a stem cell, and images of luminescence emitted by expression of the luminescent protein-coding gene (luciferase gene) in the stem cell are captured over a given period, whereby it is possible to monitor the differentiation state of a stem cell by each cell or each colony without damage to the cell.

### Brief Description of Drawings

FIG. 1 is an illustration showing an example of the method of the present invention.
FIG. 2 is a schematic diagram of hanging drop method.
FIG. 3 is a diagram showing an example of a luminescence imaging system.
FIG. 4 is microscopic images showing the result of Example 1-1 (immediately after observation) of the present invention.
FIG. 5 is microscopic images showing the result of Example 1-1 (21 hours after observation) of the present invention.
FIG. 6 is a graph showing the result of Example 1-1 of the present invention.
FIG. 7 is microscopic images showing the result of Example 1-2 (LIF added) of the present invention.
FIG. 8 is a microscopic image showing the cell selected regions in Example 1-2 (LIF added) of the present invention.
FIG. 9 is graphs showing the result of Example 1-2 (LIF added) of the present invention.
FIG. 10 is a microscopic image showing the cell selected regions in Example 1-2 (without LIF) of the present invention.
FIG. 11 is graphs showing the result of Example 1-2 (without LIF) of the present invention.
FIG. 12 is microscopic images showing the cell selected regions in Example 1-3 of the present invention.
FIG. 13 is graphs showing the result of Example 1-3 of the present invention.
FIG. 14 is microscopic images showing the result of Example 2-1 of the present invention.
FIG. 15 is a diagram schematically showing the appearance of embryoid body (EB) formation.
FIG. 16 is microscopic images showing the result of Example 2-2 of the present invention.

Mode for Carrying Out the Invention Hereinbelow, the present invention is described in detail, and the following description is intended to explain the present invention and is not intended to limit the present invention.

In one embodiment, the method for monitoring the differentiation state of a stem cell according to the present invention includes
a step (A-1) of culturing a stem cell into which a fusion gene of a promoter region of a differentiation state detection marker gene and a luciferase gene is transfected,
a step (A-2) of culturing the stem cell under a differentiation-inducing condition after the step (A-1), and
a step (A-3) of capturing images of luminescence emitted by expression of the luciferase gene in the stem cell, over at least a given period of the steps (A-1) to (A-2).

In this embodiment, the differentiation state of a stem cell subjected to differentiation induction treatment can be monitored.

In another embodiment, the method of the present invention further includes a step of subculturing the stem cell after the culture step (A-1). More specifically, in this embodiment, the method for monitoring the differentiation state of a stem cell according to the present invention includes
a step (B-1) of culturing a stem cell into which a fusion gene of a promoter region of a differentiation state detection marker gene and a luciferase gene is transfected,
a step (B-2) of further subculturing the stem cell,
a step (B-3) of culturing the stem cell under a differentiation-inducing condition after the step (B-2), and
a step (B-4) of capturing images of luminescence emitted by expression of the luciferase gene in the stem cell, over at least a given period of the steps (B-1) to (B-3).

In this embodiment, it is possible to monitor the differentiation state of a stem cell during subculture and the differentiation state of a stem cell subjected to differentiation induction treatment.

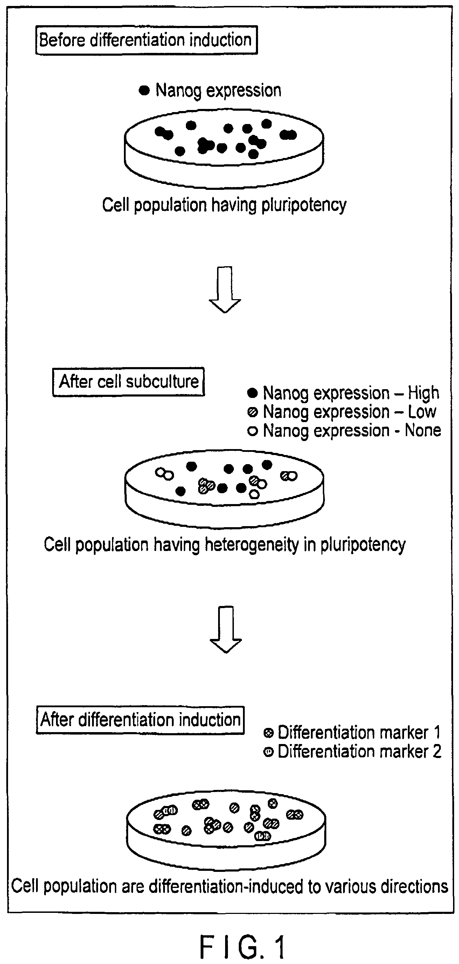

This embodiment is schematically shown in FIG. 1.

As shown in FIG. 1, when the stem cells prepared for monitoring the differentiation state are cultured under a differentiation-inhibiting condition, most of the stem cells have pluripotency and express an undifferentiation marker gene (Nanog gene) (see a dish before differentiation induction in FIG. 1). When these stem cells are cultured for a long time, some stem cells may differentiate, and thereby generating variation in pluripotency, which leads to the appearance of a cell in which expression of the undifferentiation marker gene (Nanog gene) is decreased or disappears (see a dish after cell subculture in FIG. 1). Thereafter, when the stem cells are subjected to differentiation induction treatment and cultured, some stem cells are differentiation-induced to express a specific differentiation marker gene (see a dish after differentiation induction in FIG. 1). As shown, in the present invention, expression of an undifferentiation marker gene and a differentiation marker gene can be separately detected using different type of luciferase to monitor the differentiation state of a stem cell by each cell or each colony.

In still another embodiment, the method of the present invention further includes a step of subculturing the stem cell after the culture step (A-1) but does not include the differentiation induction step (A-2). More specifically, in this embodiment, the method for monitoring the differentiation state of a stem cell according to the present invention includes
a step (C-1) of culturing a stem cell into which a fusion gene of a promoter region of a differentiation state detection marker gene and a luciferase gene is transfected,
a step (C-2) of further subculturing the stem cell, and
a step (C-3) of capturing images of luminescence emitted by expression of the luciferase gene in the stem cell, over at least a given period of the steps (C-1) to (C-2).

In this embodiment, the differentiation state of a stem cell during subculture can be monitored.

The "stem cell" herein is an arbitrary stem cell derived from mammals (for example, human, mouse, etc.), such as an embryonic stem cell (ES cell), a somatic stem cell, an induced pluripotent stem cell (iPS cell), and the like. A commercially available cell may be used as a stem cell, or a stem cell may be prepared according to a known method.

The "monitoring the differentiation state" herein refers to monitoring if the stem cell is in the undifferentiated state or to what extent the stem cell is in the differentiated state. The "differentiation state" herein includes both the state of the undifferentiated cell and the state of the differentiated cell.

The "differentiation state detection marker gene" herein includes an "undifferentiation marker gene" in which a cell specifically expresses in the undifferentiated state and a "differentiation marker gene" in which a cell specifically expresses in a specific differentiation process. In the method of the present invention, as the "differentiation state detection marker gene", either an undifferentiation marker gene or a differentiation marker gene may be used or both may be used. In addition, as the "differentiation state detection marker gene", one type may be used, or plural types (for example, 2 to 5 types) may be used. For example, in the present invention, plural types (for example, 2 or 3 types) of undifferentiation marker genes may be used, one type of an undifferentiation marker gene and one type of a differentiation marker gene may be used, or one type of an undifferentiation marker gene and plural types (for example, 2 or 3 types) of differentiation marker genes may be used.

The "undifferentiation marker gene" is an arbitrary gene in which a cell specifically expresses in the undifferentiated state, and examples thereof include Nanog, Oct, Sox, and the like. The "differentiation marker gene" is an arbitrary gene in which a cell specifically expresses in a specific differentiation process, and examples thereof include Nestin and Mashl as a neuronal differentiation marker gene and GATA4 and Nkx2.5 as a myocardial differentiation maker gene.

Hereinbelow, each step of the method of the present invention is described. In the following description, a "differentiation state detection marker gene" is also simply referred to as a "marker gene".

### 1. Preparation of Stem Cell for Monitoring

In the present invention, according to a known technology, a promoter region of a marker gene and a luciferase gene (reporter gene) are fused, and this fusion gene is introduced into a stem cell to prepare a stem cell for monitoring.

As the "promoter region of a marker gene", a known promoter region may be used, or a promoter region may be cloned based on the nucleotide sequence of a known marker gene. For example, a promoter region of Nanog is described in T. Kuroda et al., Molecular and Cellular Biology (2005 vol. 25, No. 6, p2475-2485); a promoter region of Oct is described in S. Okumura-Nakanishi et al., The Journal of Biological Chemistry (2005 vol. 280, No. 7, p5307-5317); and a promoter region of Nestin is described in L. Cheng et al., FEBS Letters, 2004, 565, p195-202.

As the "luciferase gene", a commercially available one, such as Eluc luciferase gene (green), CRB luciferase gene (red), and Renilla luciferase gene (blue) can be used. When a luciferase gene that is previously incorporated into a vector is used, it is convenient to prepare a fusion gene. Examples of a commercially available vector previously containing a luciferase gene include Eluc vector (Toyobo Co., Ltd.), CRB vector (Promega), and Renilla vector (Promega).

Herein, the present invention is described with reference to the case of using a luciferase gene as a reporter gene, but the present invention is not limited to this case, and an arbitrary "luminescent protein-coding gene" known in the art can be used as a reporter gene. The "luminescent protein-coding gene" herein is used in contradistinction to a fluorescent protein gene such as a green fluorescent protein (GFP) gene, and is especially, a gene encoding a bioluminescent protein, such as various luciferase genes known in the art.

When plural types of marker genes are used, plural types of luciferase genes are used, and each marker gene is fused with each different luciferase gene so as to be detected distinguishably. More specifically, each promoter region of the marker gene is fused with a gene encoding luciferase having a different spectral characteristic of luminescence so as to be detected distinguishably from a promoter region of other marker gene.

For example, the case of using two types of "undifferentiation marker genes", Oct-4 and Nanog genes, as marker genes is described. First, each promoter region of these undifferentiation marker genes are cloned using the gene sequences already published in an article. More specifically, each promoter specific sequence is obtained by reference to S. Okumura-Nakanishi et al., The Journal of Biological Chemistry, 2005, Vol. 280, No. 7, p5307-5317 for Oct-4 gene and T. Kuroda et al., Molecular and Cellular Biology, 2005, Vol. 25, No. 6, p2475-2485 for the Nanog gene. The obtained Nanog and Oct-4 gene promoter sequences are respectively incorporated into ELuc vector (Toyobo Co., Ltd.) and CBR vector (Promega). Eluc vector is a vector containing Eluc luciferase (green), and CBR vector is a vector containing CRB luciferase (red). Accordingly, Nanog and Oct-4 gene promoter sequences are incorporated into these vectors, to prepare undifferentiation marker expression-specific luminescent vectors pNanog-Eluc and pOct4-CRB.

A vector containing a fusion gene can be introduced into a stem cell according to a known method, such as calcium phosphate method, lipofectin method, or electroporation method. These methods can be used properly depending on the purpose or the type of the cell.

### 2. Culture Step

In the present invention, the stem cell for monitoring prepared according to the above method, more specifically, "the stem cell into which a fusion gene of a promoter region of a differentiation state detection marker gene and a luciferase gene is introduced" is cultured. Culture can be carried out on a feeder cell by adding a differentiation inhibitor necessary for the type of a stem cell to a culture medium, according to a known method. Preferably, a stem cell is cultured under a differentiation-inhibiting condition. For example, culture of mouse ES cell can be carried out on a mouse embryonic fibroblast that is a feeder cell, in DMEM medium containing leukemia inhibitory factor (LIF) that is a differentiation inhibitor, in a 35-mm dish for cell culture. The stem cell is, for example, cultured at 37.0°C for 1 to 3 days.

The stem cell may be subsequently subcultured after the above culture or may be transferred to a differentiation induction step.

Subculture can be carried out in the same condition as the above culture. In general, since a stem cell is likely to spontaneously differentiate, it is necessary to give attention to the culture condition. For example, subculture may be carried out in a culture medium containing a differentiation inhibitor, and change in the expression of undifferentiation marker may be observed to identify an undifferentiated cell. Alternatively, subculture may be carried out in a culture medium not containing a differentiation inhibitor, and change in the expression of undifferentiation marker and/or differentiation marker may be observed to identify an undifferentiated cell and/or differentiated cell.

### 3. Differentiation Induction Step

Differentiation induction of a stem cell can be carried out by culturing a stem cell under a differentiation-inducing condition according to a known method, depending on the type of the stem cell and the direction of differentiation.

The differentiation-inducing condition may be a condition for positively inducing differentiation of a stem cell or may be a condition which is not inhibiting spontaneous differentiation of a stem cell (i.e., a condition of the absence of a differentiation inhibitor).

A differentiation induction method of a stem cell includes a method by a physical stimulus, a method by a drug stimulus, a method by introduction of differentiation induction factor, and the like. Examples of the method by a physical stimulus include hanging drop method and shaking culture. Examples of the method by a drug stimulus include addition of retinoic acid to a culture medium for differentiation induction to a neural cell and addition of BMP-2 and Wnt-11 after embryoid body formation for differentiation induction to a myocardial cell.

More specifically, in differentiation induction of a stem cell, a differentiated cell may be prepared from a stem cell passing through the formation of an embryoid body by using hanging drop method or the like, or a differentiated cell may be directly prepared from a stem cell without passing through an embryoid body.

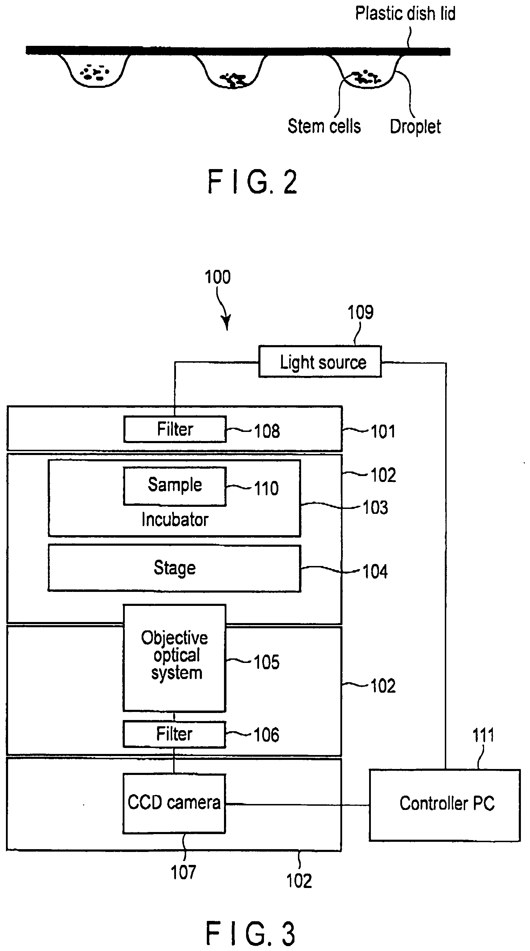

The schematic view of hanging drop method is shown in FIG. 2. In the hanging drop method, as shown in FIG. 2, a stem cell is cultured in a culture solution (droplet) hanged in the form of a waterdrop from a substrate such as a plastic dish lid. More specifically, a luminescent vector containing a promoter of an object marker gene is introduced into a cell, and the cell is seeded to a plastic dish lid for cell culture to form a droplet. After the droplet formation, 2-day continuous observation is carried out using a luminescence microscope, according to the present invention. It is possible to observe the temporal process that individual stem cells aggregate to form an embryoid body, by a luminescence microscope. As necessary, if an antibiotic-resistant gene is inserted into a luminescent vector into which a promoter of the marker gene is introduced, then it is possible to obtain an intended cell by drug selection after collecting a necessary embryoid body.

It is known that not only a cell characteristic but also interaction of each cell is involved in differentiation of an ES cell. It is also considered that the differentiation efficiency of various cells is influenced by a characteristic of each embryoid body. It is important to observe the process of forming an embryoid body since the characteristic of the embryoid body varies depending on the droplet size and the cell count to be seeded, but imaging detection during the process of forming an embryoid body has not been carried out in the present study. Another method for culturing a stem cell with shaking is also known for an embryoid body formation.

### 4. Image Capturing Step

When a luminescence image of the stem cell is captured during culture in a culture vessel, a substrate, luciferin, is added to a culture medium containing the stem cell before starting luminescence observation. The addition of luciferin may be accompanied by the addition of ATP, magnesium, and the like.

In the present invention, images of luminescence emitted by expression of a luciferase gene in the stem cell are captured over at least a given period during the culture step and the differentiation induction step. This step of capturing images can be carried out by luminescence imaging. Preferably, luminescence images are continuously captured by luminescence imaging. Luminescence imaging can be carried out using a commercially available luminescence imaging system, for example, luminescence imaging system LV200 manufactured by Olympus Corporation.

An example of the luminescence imaging system is described with reference to FIG. 3.

In Luminescence imaging system 100 shown in FIG. 3, Sample 110 containing stem cell is arranged in Incubator 103 on Stage 104. Sample 110 is usually put in a petri dish, and Stage 104 and Incubator 103 have holes for observation (not shown). In luminescence observation, a light emitted in Sample 110 proceeds through Objective optical system 105 with unnecessary light being cut in Spectral filter 106, and detected by CCD camera 107. The light detected by CCD camera 107 is sent to Controller PC 111 and made into an image. In monochromatic luminescence observation, Spectral filter 106 may not be disposed.

Light source 109 emits an illumination light for bright field observation. Spectral filter 108 is used when only a light of a specific spectrum is transmitted (fluorescence observation), and is not utilized in bright field observation.

Controller PC 111 is a computer such as a general PC, and performs control of photographing conditions of CCD camera 107, imaging and display of the obtained image, control of light amount of Light source 109, and the like. In addition, Controller PC 111 can perform image processing and image analysis, and store image analysis data together with the observation conditions.

If a part of or the whole of such device is arranged in a light shading device such as a chamber blocking the light from the outside, weak light can be accurately and stably detected without influence of light from outside. For example, as shown in FIG. 3, Sample 110 can be put in the light shading state by Dark box 102 and Lid 101.

For the optical system of luminescence imaging system, WO2006-106882 can be used as a reference.

In the present invention, continuously capturing luminescence images means that luminescence images may be sequentially captured, or luminescence images may be captured at predetermined intervals (for example, intervals of 5 minutes to 1 hour).

When plural types of marker genes are indicated distinguishably from each other by plural types of luciferase genes, as described in Example 2 set forth below, image capturing is performed with light having each spectral characteristic specific to each luciferase using a spectral filter (see Filter 106 in FIG. 3).

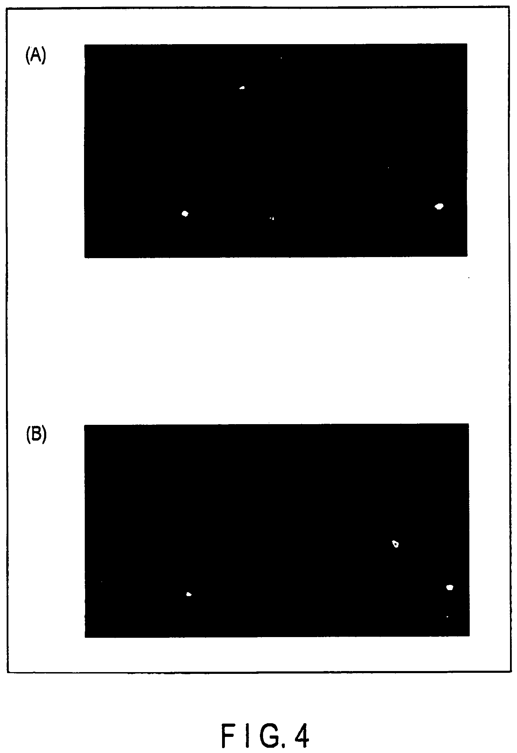

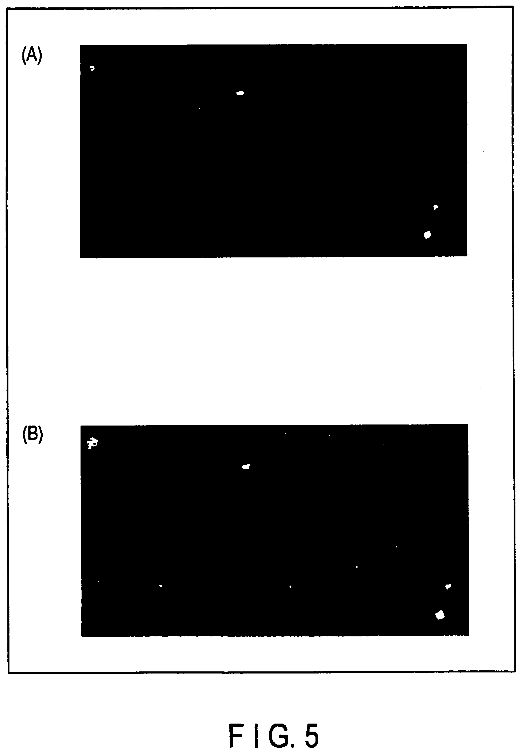

The observation of a luminescence image data may be performed in combination with the observation of a bright field image (see FIG. 4 and FIG. 5). More specifically, a bright field image is obtained in the same region where a luminescence image is obtained, whereby both expression of marker gene and morphological change of a cell by cellular differentiation (for example, morphological change of a cell by differentiation to a neural cell) can be observed.

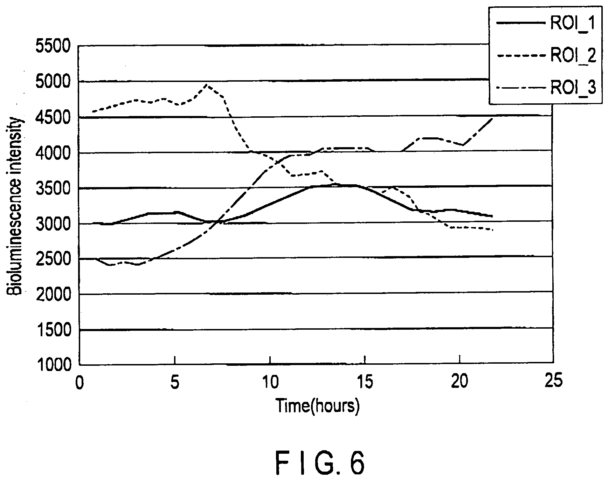

Furthermore, a luminescence intensity may be measured as an index of the differentiation state in a specific cellular region of the luminescence images obtained during the image capturing step, and a time-course change of the luminescence intensity may be analyzed (see FIG. 6).

The differentiation state of a stem cell can be identified, based on the obtained luminescence image data and/or luminescence intensity data and/or bright field image data. Preferably, the differentiation state of a stem cell can be identified, based on the time-lapse change of the luminescence image data and/or the time-lapse change of the luminescence intensity. Alternatively, preferably, the differentiation state of a stem cell can be identified, based on the data on the time-lapse change of the luminescence intensity.

Further, a stem cell showing the intended differentiation state can be collected from a stem cell population, based on the obtained luminescence image data and/or luminescence intensity data and/or bright field image data. Preferably, a stem cell showing the intended differentiation state can be obtained, based on the time-lapse change of the luminescence image data and/or the time-lapse change of the luminescence intensity. Alternatively, preferably, a stem cell showing the intended differentiation state can be obtained, based on the data on the time-course change of the luminescence intensity.

For example, it can be seen that, when the expression level of an undifferentiation marker is constantly maintained in a region of interest (ROI) containing a cell to be measured, based on the luminescence image data and/or luminescence intensity data, the cell to be measured is maintained in the undifferentiated state. On the other hand, it can be seen that, when decrease in the expression level of an undifferentiation marker is observed from the luminescence image data and/or luminescence intensity data, the cell to be measured is changed from the undifferentiated state. In this case, both decrease in the expression level of an undifferentiation marker and increase in the expression level of a differentiation marker are observed by using an undifferentiation marker and a differentiation marker together. Thereby it can be seen that the cell to be measured is oriented in the direction of differentiation even in the stage where morphological change of the cell has not yet appeared.

A pickup operation for collecting an intended stem cell may be performed under a luminescence microscope in which a luminescence image data has been obtained or may be performed after transferring the sample to an upright microscope.

Further, in the present invention, various changes are possible without limiting to the examples described above. For example, while the pickup operation is described in the above description, various cell operations including other operation such as separation of an intended cell or subcellular part in a vessel can be also applied in the present invention. In addition, in the above description, a cell, tissue or the like extracted from an individual as an *in vivo* sample is cultured in a given vessel in order to maintain the living state. Such culture shows a constitution applicable to a microscopic organism such as an embryo, microorganism, and bacterium. However, when microscopic image capturing is performed on an organism such as an experimental animal or plant (so-called, in *vivo* imaging, endoscopic observation, and the like), natural culture is performed so that stem cells and the like can be maintained in the living state *in vivo,* in the culture in a vessel using an incubator. Such culture is also included in the culture step in the present invention.

### 5. Effect of Present Invention

As described above, in the present invention, a marker gene expression level is detected as an image by luminescence imaging using luciferase as a reporter, whereby it is possible to measure the luminescence intensity from each stem cell or colony and identify the differentiation state by each stem cell or colony. Stem cell imaging is performed using luciferase as a reporter, whereby it is possible to quantitatively observe the time-course expression of a marker gene while maintaining the cell in the living state. Stem cell imaging is performed using luciferase as a reporter, whereby it is possible to monitor the extent of cell differentiation that cannot be identified by the morphological change of a cell, and it is possible to understand the differentiation state more precisely than by the conventional method. Image detection by stem cell imaging is carried out to observe the time-course data of each stem cell or colony, whereby more precise detection is possible in consideration of the stage of cell cycle.

Detection using a combination of plural types of undifferentiation and differentiation markers, not one type, is carried out, whereby the stage of the differentiation state can be more accurately understood.

In addition, bright field observation for morphological observation is used together with luminescence imaging excellent in quantitative property, whereby it is possible to also capture morphological change of a cell and marker gene expression without irradiating excitation light on a cell.

While the mechanism of maintaining undifferentiation of a stem cell is yet unclear, gene expression change of various undifferentiation markers can be also detected by utilizing the method of the invention, where the gene expression change are not found by the morphological change. For example, the expression levels of the Oct-4 and Nanog genes, which have been already identified, are visualized by a stem cell or colony, whereby expression proportion of each undifferentiation marker can be understood. As a result, how the undifferentiated state is maintained can be investigated, and the effect of the expression level of the undifferentiation marker on the direction of differentiation can be investigated in the cell with the same morphology.

### Examples

### Example 1: Examples using undifferentiation marker

### Example 1-1: Observation and analysis of transient expression cells

### (1) Preparation of ES cells into which a fusion gene of a promoter region of an undifferentiation marker gene and a luciferase gene is introduced

For cloning of Nanog gene promoter region, the information of promoter sequence was obtained with reference to Non-Patent Literature "T. Kuroda et al., Molecular and Cellular Biology, 2005, Vol. 25, No. 6, pp. 2475-2485".

Nanog gene promoter region sequence was obtained using a mouse genomic DNA as a template. As a primer for amplifying Nanog gene promoter region, the following primers were used.
forward primer: CTACTCGAGATCGCCAGGGTCTGGA (SEQ ID NO: 1)
reverse primer: CTACTCGAGCGCAGCCTTCCCACAGAAA (SEQ ID NO: 2)

The obtained Nanog gene promoter sequence was inserted into pGL4-basic vector (Promega) to prepare "Nanog gene expression-specific luminescent vector pNanog-GL4".

Feeder cells were prepared to culture ES cells. Specifically, a 35-mm plastic dish was coated with 0.1% gelatin solution and washed with PBS three times. MEF cells (mouse embryonic fibroblast) were treated with mitomycin C to stop cell division, seeded in the dish coated with gelatin, and cultured overnight. As the culture medium, DMEM (containing phenol red and 10% FCS) was used. The next day, mouse ES cells (BRC6 strain, Riken BRC) were seeded on the feeder cells in the 35-mm dish.

After overnight culture, the "pNanvg-GL4 gene expression vector" was transfected into the mouse ES cells, and the transfected ES cells were cultured overnight using DMEM containing 15% KSR (Knockout Serum [Gibco]) and LIF (leukemia inhibitory factor) as a culture medium. For the gene transfection, Nucleofection method using Amaxa Nucleofector (Wako Pure Chemical Industries, Ltd.) was performed. The next day, the culture medium was replaced with DMEM containing HEPES (15% KSR, without phenol red).

Although a transiently transfected cell line is used in the present experiment, a stably transfected cell line into which a drug resistance gene is inserted to select with a drug may be used.

### (2) Observation and analysis of ES cells

D-luciferin (manufactured by Promega KK: final concentration of 100 µM) was added for luminescence observation, and the mouse ES cells were observed using a luminescence microscope LV200 (manufactured by Olympus Corporation). As the luminescence observation conditions, the luminescence images of the mouse ES cells were taken under 15 min exposure at 45 minutes intervals, thereby observing Nanog gene expression level. Imaging was performed using 20x objective lens and CCD camera of ImagEM (manufactured by Hamamatsu Photonics K.K.) with 1×1 binning.

After the time-lapse observation, the captured observation images were saved, and numerical data was analyzed from the observation images using an image analysis software "AQUACOSMOS (manufactured by Hamamatsu Photonics K.K.)", and the analyzed numerical data was shown graphically.

The obtained luminescence images are shown in FIGS. 4 and 5. FIG. 4 (A) shows a luminescence image immediately after luminescence observation by LV200, and FIG. 4 (B) shows a superimposed image of a bright field image and luminescence image immediately after luminescence observation by LV200. FIG. 5 is an image at 21 hours after observation. FIG. 5 (A) shows a luminescence image, and FIG. 5 (B) shows a superimposed image of a bright field image and a luminescence image.

Three ES cell colonies were selected, and numeric conversion of the luminescence intensity was performed in each region. The result is shown in FIG. 6.

The selected three regions are shown in FIGS. 4 and 5, and each is referred to as ROI 1 to 3. Based on the image data of FIGS. 4 and 5, it can be seen that, at 21 hours after observation, the luminescence intensity is decreased in the region of ROI 2, and the luminescence intensity is increased in the region of ROI 3. This result is also consistent with the result of the luminescence intensity of FIG. 6.

From the results of FIGS. 4 to 6, a colony with increased luminescence (ROI 3), a colony with few change of luminescence (ROI 1) and a colony with decreased luminescence (ROI 2) from the start of culture, could be seen, and it was found that the pattern of Nanog gene expression as an entire colony was different depending on each stem cell colony. The pattern of the gene expression refers to the change in gene expression with the lapse of time. ES cells are known to be a heterogenous cell population having a different characteristic, and it is consistent with conventional understanding. It could be seen from the results that the differentiation state of ES cells could be monitored by each cell or colony.

From the result of the experiment, it could be seen that gene expression of an undifferentiation marker Nanog could be continuously measured by using the luminescence intensity of luciferase as a reporter.

While it is an observation for 21 hours or so in the present study, in a case where a long term observation is performed over several days, observation can also continuously be performed by replacing with an ES cell culture solution to which luciferin and a differentiation inhibitor LIF (leukemia inhibitory factor) are added. If necessary, feeder cells may be also added.

### Example 1-2: Long term observation and analysis of stable expression cells

In the present example, a vector containing a drug resistance gene is used as a gene transfer vector, cells into which gene transfer was performed (stable expression cells) were selected by drug selection, and a long term observation and analysis were performed. The detail of the present example is set forth below.

### (1) Preparation of ES cells into which a fusion gene of a promoter region of an undifferentiation marker gene and a luciferase gene is introduced

For cloning of Nanog gene promoter region, the promoter sequence was obtained with reference to Non-Patent Literature "T. Kuroda et al., Molecular and Cellular Biology, 2005, Vol. 25, No. 6, pp. 2475-2485".

Nanog gene promoter region sequence was obtained using a mouse genomic DNA as a template. As a primer for amplifying Nanog gene promoter region, the following primers were used.
forward primer: CTACTCGAGATCGCCAGGGTCTGGA (SEQ ID NO: 1)
reverse primer: CTACTCGAGCGCAGCCTTCCCACAGAAA (SEQ ID NO: 2)

In the gene transfer vector, a luciferase gene part of a neomycin-resistant pGL4 vector (Promega) was replaced with Eluc luciferase gene. The obtained Nanog gene promoter sequence was inserted into the vector to prepare "Nanog gene expression-specific luminescent vector pNanog-Eluc", and transfection into mouse ES cells was performed. For the gene transfection, Nucleofection method using Amaxa Nucleofector (Wako Pure Chemical Industries, Ltd.) was performed. The gene-transferred cells were selected by adding a drug G418 to prepare a stable expression cell line.

Feeder cells were prepared in order to culture ES cells. Specifically, a 35-mm plastic dish was coated with 0.1% gelatin solution and washed with PBS three times. MEF cells (mouse embryonic fibroblast) were treated with mitomycin C to stop cell division, seeded in the dish coated with gelatin, and cultured overnight. As the culture medium, DMEM (containing phenol red and 10% FCS) was used. The next day, mouse ES cells (BRC6 strain, Riken BRC) were seeded on the feeder cells in the 35-mm dish.

After overnight culture, mouse ES cells which constitutively express Nanog-Eluc were cultured overnight using DMEM containing 15% KSR (Knockout Serum [Gibco]) and LIF (leukemia inhibitory factor) as a culture medium. After the culture, in order to perform a luminescence observation in the two conditions of "LIF added" and "without LIF", the culture medium was each replaced with DMEM containing LIF and HEPES (without 15% KSR or phenol red) and DMEM containing HEPES (without 15% KSR or phenol red, and without LIF), on the next day of transfection. The addition of LIF was carried out by using the product name of "LIF Human, recombinant, Culture Supernatant" (manufactured by Wako Pure Chemical Industries, Ltd.) in a conventional concentration.

Although a stable expression cell line into which a drug resistance gene is inserted to select with a drug is used in the present experiment, a transient expression cell line may be used.

### (2) Observation and analysis of ES cells

D-luciferin (manufactured by Promega KK: final concentration of 500 µM) was added for luminescence observation, and the mouse ES cells were observed using a luminescence microscope LV200 (manufactured by Olympus Corporation). As the luminescence observation conditions, the luminescence images of the mouse ES cells were taken under 12 min exposure at 15 minutes intervals, thereby observing Nanog gene expression level. Imaging was performed using 20x objective lens and CCD camera of ImagEM (manufactured by Hamamatsu Photonics K.K.) with 1×1 binning.

After the time-lapse observation, the captured observation images were saved, and numerical data was analyzed from the observation images using an image analysis software "AQUACOSMOS (manufactured by Hamamatsu Photonics K.K.)", and the analyzed numerical data was shown graphically.

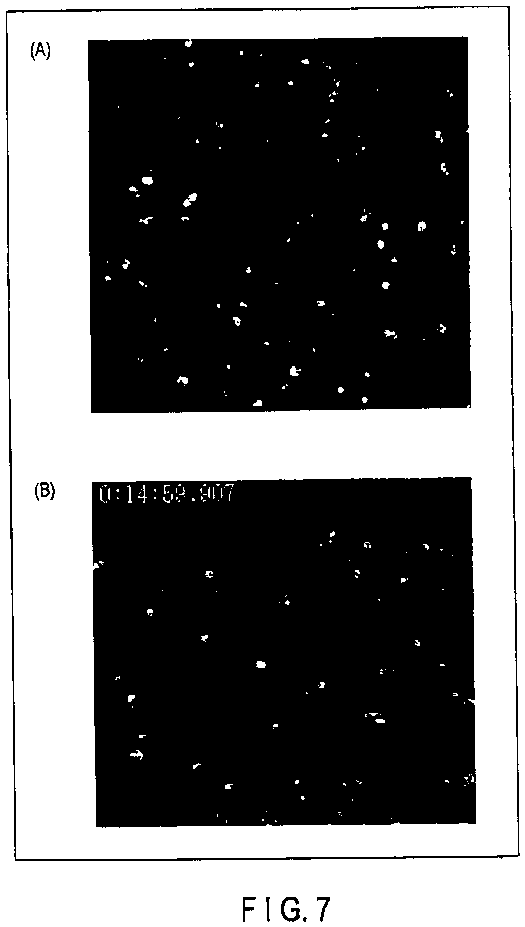

An experiment was performed by observing cells in the culture medium containing LIF, and the obtained luminescence images are shown in FIG. 7. FIG. 7A shows a luminescence image immediately after luminescence observation by LV200, and FIG. 7B shows a superimposed image of a bright field image and luminescence image immediately after luminescence observation by LV200. The luminescence images were shown by pseudo-color yellow.

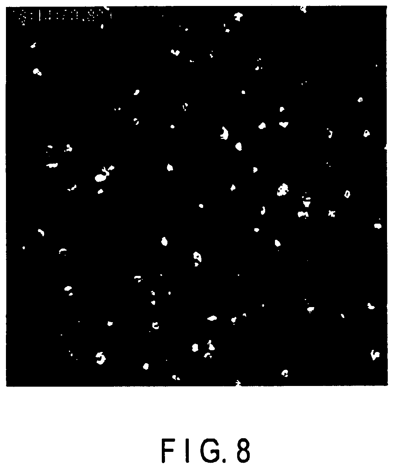

In order to quantify the Nanog expression level, ES cell colonies were selected at multiple sites, and numeric conversion of the luminescence intensity was performed. The cell selection regions are shown in FIG. 8.

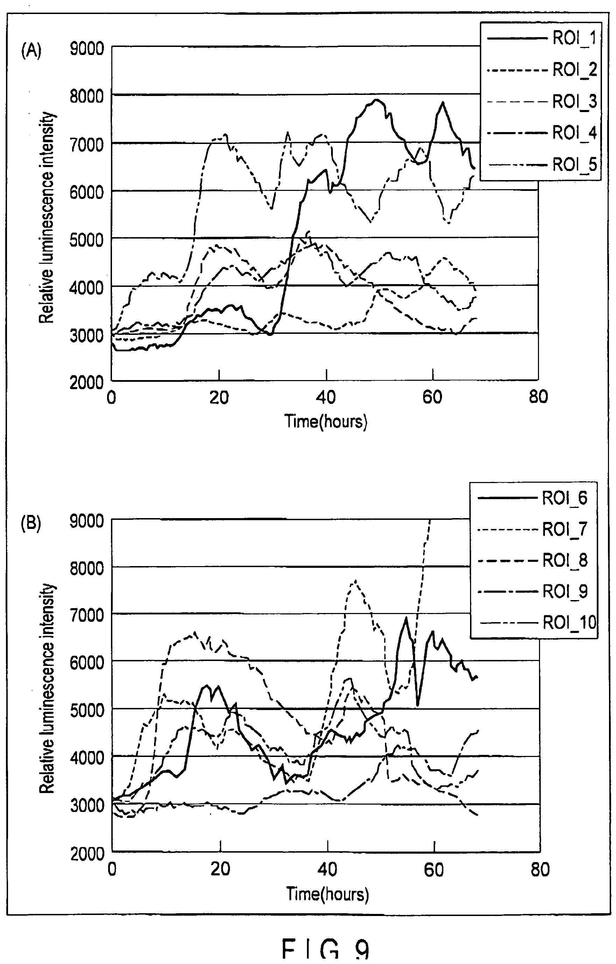

Regarding 10 colonies of ROI 1 to 10 among the selected regions, the numerical values of the luminescence intensity were shown graphically, and shown in FIG. 9. Each is referred to as ROI 1 to 10. FIG. 9A shows the results of ROI 1 to 5, and FIG. 9B shows the results of ROI 6 to 10.

Based on the results of FIG. 9, it was observed that the Nanog gene expression level varied in many colonies. It can be also seen that there is 16 to 18-hour cycle oscillation. An oscillation phenomenon in stem cells was reported in Chambers I., et al., Nature, Vol. 450, pp. 1230-1234, 2007, but there has never been a research which performs Nanog expression analysis by each colony over an extended period.

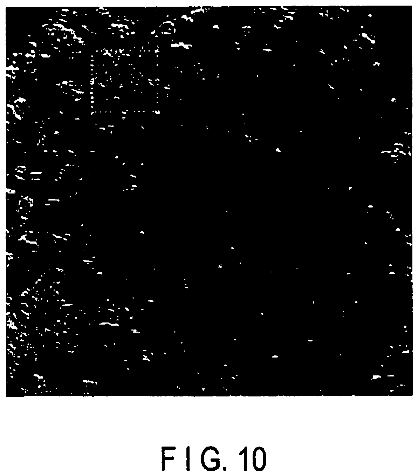

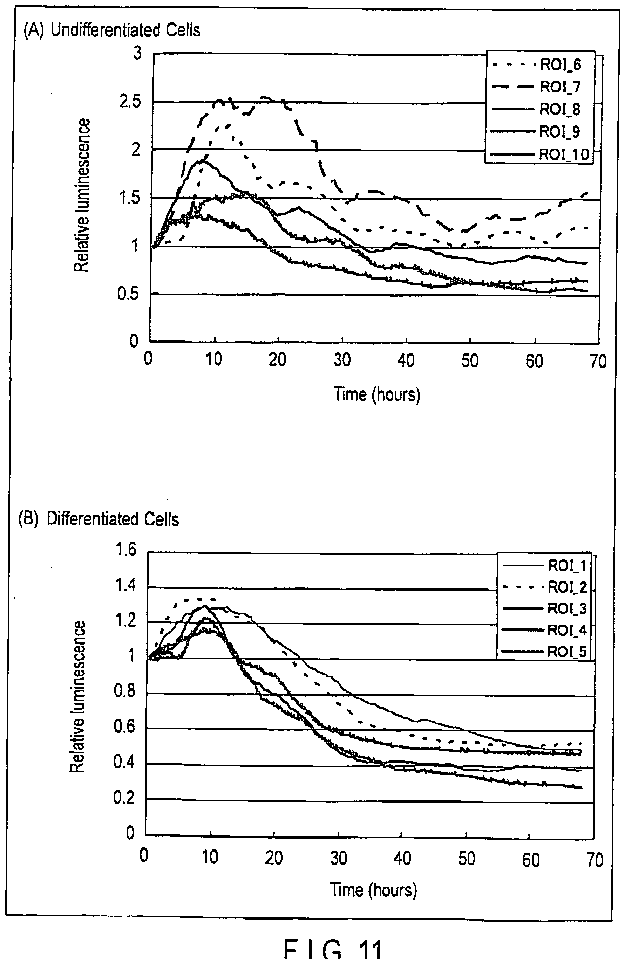

Next, luminescence observation and bright field observation were performed in the condition where LIF was removed from the culture medium. In order to quantify the Nanog expression level, ES cell colonies were selected at multiple sites, and numeric conversion of the luminescence intensity was performed. The colonies were classified into colonies in the undifferentiated state and somewhat broadened colonies in the differentiated state by utilizing morphological information based on the bright field observation, and the numeric conversion of the luminescence intensity was performed. The cell selection regions are shown in FIG. 10. Undifferentiated cells were selected as ROI 6 to 10, and differentiated cells were selected as ROI 1 to 5. The numerical values of the luminescence intensity of the selected undifferentiated cells and differentiated cells were shown graphically, and shown in FIG. 11. FIG. 11A shows the result of undifferentiated cells, and FIG. 11B shows the result of differentiated cells. Comparing luminescence patterns based on the morphological classification, oscillation was seen in the colonies in the undifferentiated state, but oscillation was likely to disappear in the colonies where differentiation had proceeded.

### Example 1-3: Long term observation and analysis of stable expression cells under drug stimulus

Mouse ES cells used in Example 1-2 were used, which is constitutively expressing Nanog-Eluc. The cells were differentiation-induced with FGF (Fibroblast Growth Factor), and then observed under condition with signal transduction inhibitor. FGF signal is a signal transduction which induces stem cell differentiation, and it has been reported that the stem cell returns from the differentiated state to the undifferentiated state by the action of an inhibitor (T. Kunath et al., Development 134, pp. 2895-2902, 2007). The preparation method of ES cells is in the same manner as in Example 1-2.

### (1) Preparation of ES cells

After seeding in a dish and culturing overnight, the mouse ES cells constitutively expressing Nanog-Eluc were cultured overnight with DMEM culture medium containing 15% KSR (Knockout Serum [Gibco]) and LIF (leukemia inhibitory factor). After the culture, the culture medium was replaced with DMEM containing HEPES (without 15% KSR or phenol red, without LIF).

### (2) Observation and analysis of ES cells

D-luciferin (manufactured by Promega KK: final concentration of 500 µM) was added for luminescence observation, FGF (FGFβ human, PeproTech Inc.) was added so as to have a final concentration of 10 ng/ml, and then the mouse ES cells were observed using a luminescence microscope LV200 (manufactured by Olympus Corporation). As the luminescence observation conditions, the luminescence images of the mouse ES cells were taken under 55 min exposure at 1 hour intervals, thereby observing Nanog gene expression level. After 40 hours observation, PD184352 (manufactured by Wako Pure Chemical Industries, Ltd.) and SU5402 (manufactured by Wako Pure Chemical Industries, Ltd.) that are inhibitors of FGF signaling pathway were added thereto, and observation was carried out for additional 26 hours.

PD184352 is an inhibitor of ERK pathway, SU5402 is a FGF-R tyrosine kinase inhibitor, and it has been reported that these inhibitor are used in combination, thereby increasing the inhibitory effect on FGF signal. The final concentration of these inhibitor was 10 mM and 2 mM, respectively. Imaging was performed using 20x objective lens and CCD camera of ImagEM (manufactured by Hamamatsu Photonics K.K.) with 1×1 binning.

After the time-lapse observation, the captured images were saved, and numerical data was analyzed from the observation images using an image analysis software "AQUACOSMOS (manufactured by Hamamatsu Photonics K.K.)", and the analyzed numerical data was shown as graph data.

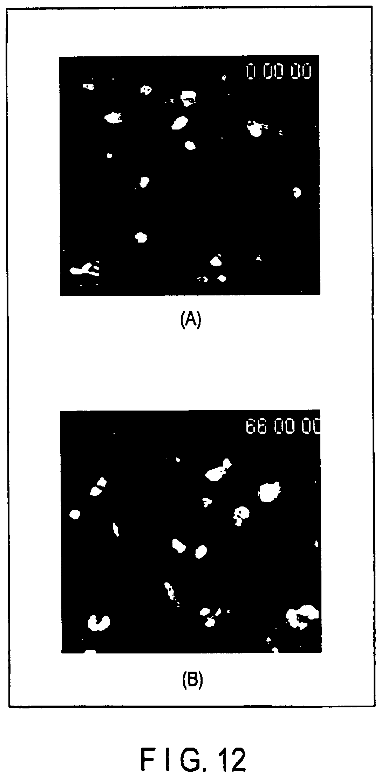

In order to quantify the Nanog expression level, ES cell colonies were selected at multiple sites. A part of the cell selection region of the luminescence images is shown in FIG. 12. FIG. 12A is a luminescence image at the beginning of the luminescence observation by LV200, and FIG. 12B is a luminescence image at the end of the luminescence observation by LV200.

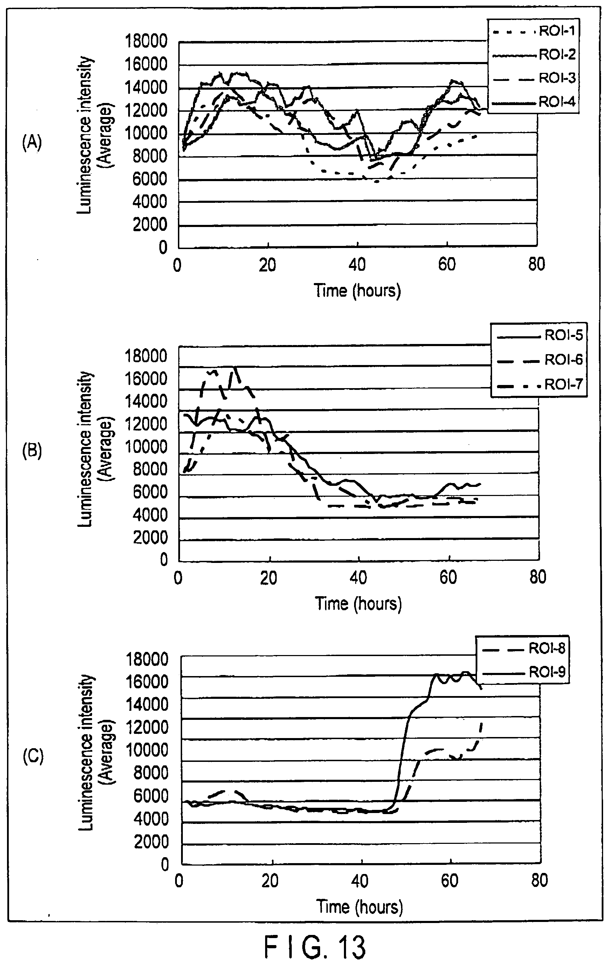

The luminescence intensities of the selected 9 regions ROI 1 to 9 were shown graphically, and shown in FIG. 13. The Nanog expression levels of colonies were classified into three types of expression patterns A to C. The expression patterns A to C are shown in FIGS. 13A to 13C, respectively.

In the expression pattern A, the Nanog expression was increased by the addition of the inhibitor 40 hours after when the Nanog expression was decreased. It is considered that the expression of the undifferentiation marker Nanog was increased by inhibiting differentiation induction. It is consistent with a report that the Nanog expression is reversible.

In the expression pattern B, the Nanog expression remained decreased even after adding the inhibitor. It is considered that the effect of the inhibitor was not seen, because differentiation induction was proceeding.

In the expression pattern C, the Nanog expression was not seen when adding FGF, but the Nanog expression was increased by the addition of the inhibitor. While physiological significance is unclear, it could be observed that the pattern of the Nanog expression was different by each colony, as shown in these classifications.

It could be seen from these results that the pattern of the Nanog gene expression, such as the Nanog gene expression level and duration of fluctuation, varied depending on each stem cell colony. ES cells are known to be a heterogenous cell population having a different characteristic, and it is consistent with conventional understanding. It could be seen from the result that the differentiation state of ES cells could be monitored by each cell or colony. As described above, continuous gene expression pattern analysis is performed by each cell, whereby more precise analysis is possible, as is not in detection and quantification of a pluripotent differentiation marker at the end point such as a flow cytometer.

After analyzing by each cell or colony in detail, an intended cell is obtained using the luminescence intensity showing gene expression level and a luminescence pattern showing gene expression pattern as indexes, whereby it is possible to easily purify the stem cells. The obtained stem cells are further subcultured, whereby it may reveal a characteristic of each colony.

Based on the result of the experiment, it could be seen that gene expression of an undifferentiation marker Nanog can be continuously measured using the luminescence intensity of luciferase as a promoter.

### Example 2: Examples of analysis using undifferentiation marker and differentiation marker

### Example 2-1: Observation of ES cells before differentiation induction

In the present example, undifferentiation marker Nanog gene and neural differentiation marker Nestin gene are detected using green (Eluc luciferase) and red (CBR luciferase) that have a different spectral characteristic as reporters. In the present example, when stem cells are differentiation-induced to neural cells, the change of each marker gene expression associated with differentiation induction of ES cells is detected and quantified by luminescence imaging.

### (1) Preparation of ES cells into which both a fusion gene of a promoter region of an undifferentiation marker gene with a luciferase gene and a fusion gene of a promoter region of a differentiation marker gene with a luciferase gene are transfected.

For cloning of promoter regions of Nanog gene and Nestin gene, the gene sequences already published in an article were used. The promoter sequences were obtained with reference to Non-Patent Literature "T. Kuroda et al., Molecular and Cellular Biology, 2005, Vol. 25, No. 6, pp. 2475-2485" for Nanog gene, and Non-Patent Literature "L. Cheng et al., FEBS Letters, 2004, 565, pp. 195-202 for Nestin gene.

Nanog gene promoter region sequence was obtained using a mouse genomic DNA as a template. As a primer for amplifying the Nanog gene promoter region, the following primers were used.
forward primer: CTACTCGAGATCGCCAGGGTCTGGA (SEQ ID NO: 1)
reverse primer: CTACTCGAGCGCAGCCTTCCCACAGAAA (SEQ ID NO: 2)

Nestin gene promoter region sequence was obtained using a mouse genomic DNA as a template. As a primer for amplifying the Nestin gene promoter region, the following primers were used.
forward primer: GAGAACGCGTGGGCTGTGTGTTGCACT (SEQ ID NO: 3)
reverse primer: GAGACTCGAGGTGGAGCACTAGAGAAGGGAGT (SEQ ID NO: 4)

The obtained promoter sequences of Nanog and Nestin genes were inserted into ELuc vector (Toyobo Co., Ltd.) and CBR vector (Promega), respectively, to prepare "undifferentiation marker expression-specific luminescent vector pNanog-Eluc" and "differentiation marker expression-specific luminescent vector pNestin-CBR" into which different types of luciferase were incorporated.

Feeder cells were prepared in order to culture ES cells for transfection. Specifically, a 35-mm plastic dish was coated with 0.1% gelatin solution and washed with PBS three times. MEF cells (mouse embryonic fibroblast) were treated with mitomycin C to stop cell division, seeded in the dish coated with gelatin, and cultured overnight. As the culture medium, DMEM (containing phenol red and 10% FCS) was used. The next day, mouse ES cells (BRC6 strain, Riken BRC) were seeded on the feeder cells in the 35-mm dish.

After overnight culture, two vectors pNanog-Eluc and pNestin-CBR were transfected into mouse ES cells, and the transfected cells were cultured overnight using DMEM containing 15% KSR (Knockout Serum ([Gibco]) and LIF (leukemia inhibitory factor) as a culture medium. For the gene transfection, Nucleofection method with Amaxa Nucleofector (Wako Pure Chemical Industries, Ltd.) was used. The next day, the culture medium was replaced with DMEM containing HEPES (without 15% KSR or phenol red), and 1 µM retinoic acid (Sigma) was added thereto for differentiation induction into neural cells.

### (2) Observation of ES cells

D-luciferin (manufactured by Promega KK: final concentration of 100 µM) was added for luminescence observation, and the mouse ES cells were observed using a luminescence microscope LV200 (manufactured by Olympus Corporation). In order to divide optical spectra derived from two types of luciferase having a different spectral characteristic, images were captured by each luminescence using two types of spectral filters BP515-560 (Sigma) and 610ALP (Sigma).

At 48 hours after transfection, observation was performed using LV200. Multicolor detection of green and red was performed using the above spectral filters. The observation conditions are as follows.

Observation apparatus: LV200 (Olympus), Objective lens: 10 times (NA 0.45), CCD camera: ImagEM (Hamamatsu Photonics)

Exposure time: Filter: 515-560 3 min, Filter: 610ALP 3 min, EM gain: 1200

Culture medium for observation: Culture medium for mouse ES cells containing 25 mM HEPES and 500 µM D-Luciferin (Wako)

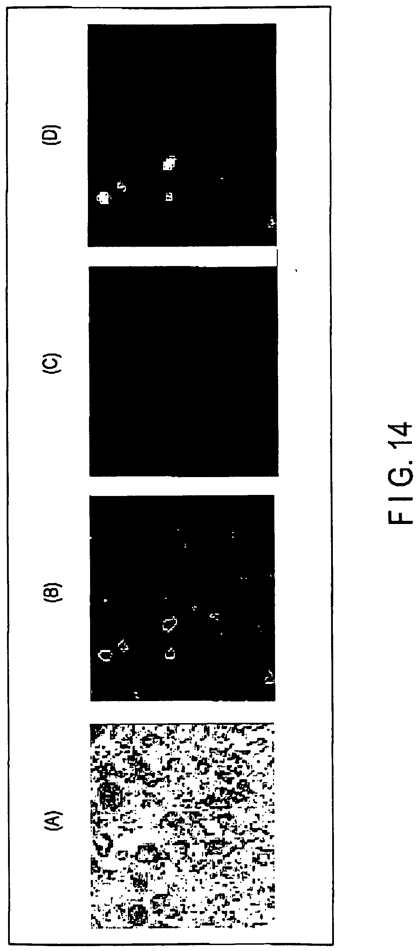

The result of capturing images at 48 hours after transfection is shown in FIG. 14. FIG. 14A shows a bright field image, FIG. 14B shows a luminescence image 1 (Nanog expression) using a filter BP515-560, FIG. 14C shows a luminescence image 2 (Nestin expression) using a filter 610ALP, and FIG. 14D shows a superimposed image of luminescence images 1 and 2. The exposure time in each observation was set to 3 minutes, and luminescence detection by each colony was possible. It is considered that the undifferentiated state can be maintained since culture is performed in the condition of LIF addition, but the expression of Nestin is slightly seen.

As described above, according to the present example, it can be seen that the state from the undifferentiated state to the state after differentiation can be sequentially monitored for an individual colony. Particularly, it can be seen that each gene expression can be simultaneously monitored in a colony in which both the undifferentiated state and the state after differentiation are mixed. In addition, it can be seen that, also for a plurality of colonies, colonies in the undifferentiated state and colonies after differentiation can be simultaneously monitored. The method of the present invention can simultaneously monitor the different states, thereby providing a novel application which overcomes a problem of crosstalk between different excitation light in fluorescence observation in the field of regenerative medicine.

### Example 2-2: Observation and analysis of ES cells after differentiation induction

In order to induce the differentiation of ES cells, an embryoid body (EB) is generally formed in a suspension culture system. While a plurality of the preparation methods such as hanging drop method is known, in the present study, an embryoid body is formed using EZ BindShut (Iwaki) coated with special phospholipid polymer in the culture surface. The Nestin gene expression is detected in the embryoid body where differentiation has proceeded.

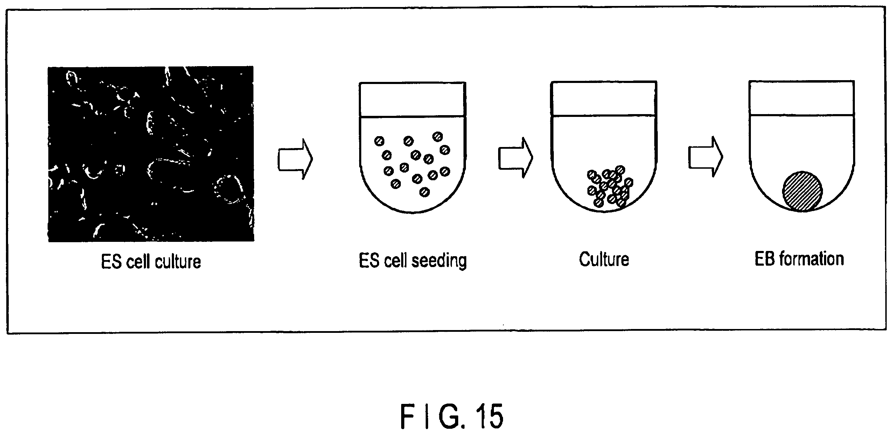

The preparation of an embryoid body (EB) was carried out as follows.
(1) A 35-mm dish of mouse ES cells in the confluent culture state were prepared.
(2) The mouse ES cells were washed once with PBS, and then subjected to trypsin treatment.
(3) The cell count was adjusted so as to be finally 200 µL/well (1000 cells/well), and transfection was performed by Nucleofection.
(4) The cells were seeded in DMEM containing 15% KSR (Knockout Serum (Gibco)), and added to a low adhesive U-bottom microplate (EZ BindShut, AGC TECHNO GLASS CO., LTD.).
(5) Culture was performed in an incubator for a cell. The culture initiation date was defined as day 0 of embryoid body (EB) formation. The appearance of embryoid body (EB) formation is schematically shown in FIG. 15.
(6) When performing luminescence observation, the embryoid body was collected using a micro pipette and transferred to a 35-mm dish containing a culture medium for luminescence observation (i.e., DMEM containing HEPES (without 15% KSR or phenol red)) to which D-luciferin (Promega, final concentration of 500 µM) was added.
(7) Luminescence observation was performed using LUMINOVIEW (LV200, Olympus).

The observation conditions are as follows.

Observation apparatus: LV200 (Olympus), Objective lens: 10× folds (NA 0.45), CCD camera: ImagEM (Hamamatsu Photonics), EM gain: 1200

Culture medium for observation: Culture medium for mouse ES cells containing 25 mM HEPES and 500 µM D-Luciferin (Promega)

The observation images captured at day 2 and day 12 after embryoid body (EB) formation were saved, and numerical data analysis was performed from the observation images using an image analysis software "AQUACOSMOS (manufactured by Hamamatsu Photonics K.K.)". Based on the luminescence intensity data obtained by dividing spectrum using each spectral filter, a luminescence intensity derived from each luciferase was calculated by the calculation technique used in the conventional fluorescence observation, and the gene expression intensity and expression ratio of each marker were analyzed.

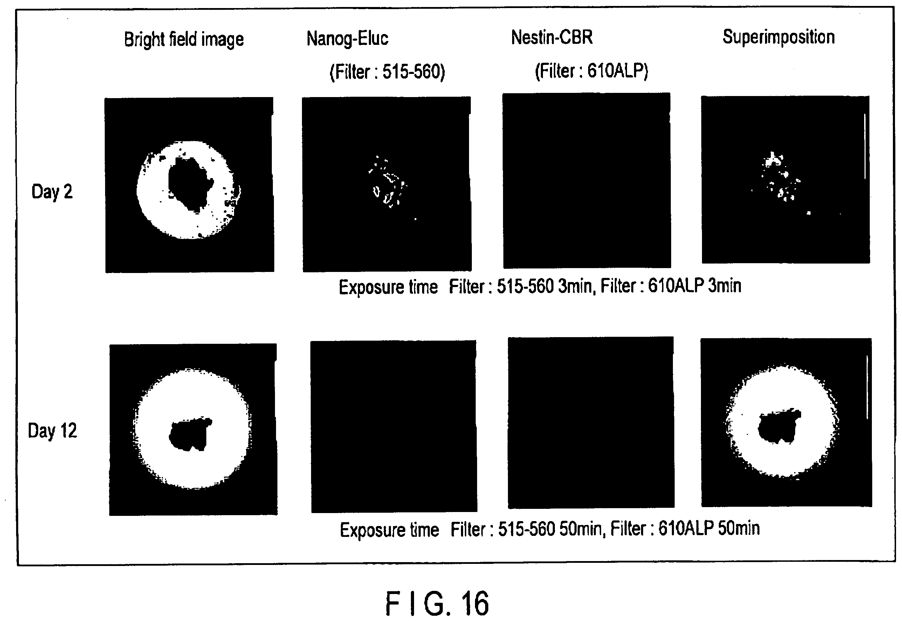

The observation images at day 2 and day 12 after embryoid body (EB) formation are shown in FIG. 16. In the upper row in FIG. 16, there are provided images on day 2 after embryoid body (EB) formation, showing, starting from the left, a bright field image, luminescence image 1 using filter BP515-560 (Nanog expression), luminescence image 2 using filter 610ALP (Nestin expression), and a superimposed image of luminescence images 1 and 2. The lower row in FIG. 12 shows images on day 12 after embryoid body (EB) formation and, starting from the left, a bright field image, luminescence image 3 using filter BP515-560 (Nanog expression), luminescence image 4 using filter 610ALP (Nestin expression), and a superimposed image of luminescence images 3 and 4.

The analysis result is shown below.

Luminescence intensity in embryoid body (average signal intensity - background numerical intensity):
Day 2 after EB formation (transfection)
Eluc: 3394, CBR: 1062, Eluc/CBR = 3.19
Day 12 after EB formation (transfection)
Eluc: 105, CBR: 3726, Eluc/CBR = 0.03

The expression levels of Nanog and Nestin were compared on day 2 and day 12 after EB formation. On day 2, ELuc expression was high and Nanog expression was dominant (Eluc/CBR = 3.19). On day 12 after EB formation, Nanog expression decreased and Nestin expression increased (Eluc/CBR = 0.03). It was found that Nanog expression decreased and Nestin expression increased, as differentiation induction proceeded with time-course analysis.

In the present study, the results of observing an embryoid body at day 2 and day 12 after differentiation induction were compared. It is also possible to sequentially observe under a microscope as long as the microscope has a function to culture cells. In the case of that a long term observation is difficult, it may be observed in the early stage, the middle stage and the late stage of sequential observation of differentiation induction. A transient gene expression cell is used in the present study, but an experiment may be conducted using a gene-transferred stable cell line. In the present experiment, a bright field observation is combined with the luminescence observation, whereby the morphological change can be captured when the cell differentiates to a neural cell, and a morphological information can be obtained at the timing when expression of a marker gene are not observed. Thus, it is possible to carry out a more precise experiment.

It is shown from these results that stem cells having various characteristics or differentiated cells derived from stem cells are present in the differentiation-induced state, and it is necessary to select a colony meeting the object of the experiment from them. Images are captured using luminescence as an index and the gene expression level is recognized as image information, whereby it is possible to monitor the extent of cell differentiation, by each stem cell, which cannot be identified by the morphological change of a cell.

In the above-described description, in order to select a colony or stem cells as a region (ROI) necessary for image processing, a superimposed image of a luminescence image and a bright field image may be displayed on a PC display or a separate display, or only a luminescence image may be displayed. It is preferable that a bright field image is used together with a luminescence image, when a contour information as morphological information by each stem cell or by each colony to which the stem cell belongs is necessary, or a site information such as distribution of stem cells in a colony is necessary. When limited to the purpose of obtaining the information of the contour and/or site, a fluorescence image obtained by performing fluorescence observation, in place of a bright field image, may be superimposed with the luminescence image, or a luminescence image showing the gene expression level may be further superimposed on a morphological image obtained by superimposing both a bright field image and a fluorescence image.

## Claims

1. A method for monitoring the differentiation state of each stem cell in a colony of stem cells, comprising:
a step (A-1) of culturing stem cells into which a fusion gene of a promoter region of a differentiation state detection marker gene and a luciferase-coding gene is transfected;
a step (A-2) of culturing the stem cells under a differentiation-inducing condition after the step (A-1); and
a step (A-3) of capturing images of luminescence emitted by expression of the luciferase-coding gene in the stem cells, over at least a given period of the steps (A-1) to (A-2), to obtain luminescence images indicating the luciferase-coding gene expression level of each stem cell in the colony,
wherein the stem cell is a stem cell into which plural types of fusion genes are transfected,
each fusion gene contains a promotor region of a different type of a differentiation state detection marker gene,
each promotor region of the differentiation state detection marker gene is fused with a gene encoding each luciferase having a different spectral characteristic of luminescence, so as to be detected distinguishably from a promotor region of other differentiation state detection marker gene, and
the image capturing step is a step of capturing each luminescence image corresponding to a spectral characteristic specific to each luciferase to obtain plural luminescence images.

2. A method for monitoring the differentiation state of each stem cell in a colony of stem cells, comprising:
a step (B-1) of culturing stem cells into which a fusion gene of a promoter region of a differentiation state detection marker gene and a luciferase-coding gene is introduced;
a step (B-2) of further subculturing the stem cells;
a step (B-3) of culturing the stem cells under a differentiation-inducing condition after the step (B-2); and
a step (B-4) of capturing images of luminescence emitted by expression of the luciferase-coding gene in the stem cells, over at least a given period of the steps (B-1) to (B-3), to obtain luminescence images indicating the luciferase-coding gene expression level of each stem cell in the colony,
wherein the stem cell is a stem cell into which plural types of fusion genes are transfected,
each fusion gene contains a promotor region of a different type of a differentiation state detection marker gene,
each promotor region of the differentiation state detection marker gene is fused with a gene encoding each luciferase having a different spectral characteristic of luminescence, so as to be detected distinguishably from a promotor region of other differentiation state detection marker gene, and
the image capturing step is a step of capturing each luminescence image corresponding to a spectral characteristic specific to each luciferase to obtain plural luminescence images.

3. A method for monitoring the differentiation state of each stem cell in a colony of stem cells, comprising:
a step (C-1) of culturing stem cells into which a fusion gene of a promoter region of a differentiation state detection marker gene and a luciferase-coding gene is introduced;
a step (C-2) of further subculturing the stem cells; and
a step (C-3) of capturing images of luminescence emitted by expression of the luciferase-coding gene in the stem cells, over at least a given period of the steps (C-1) to (C-2), to obtain luminescence images indicating the luciferase-coding gene expression level of each stem cell in the colony,
wherein the stem cell is a stem cell into which plural types of fusion genes are transfected,
each fusion gene contains a promotor region of a different type of a differentiation state detection marker gene,
each promotor region of the differentiation state detection marker gene is fused with a gene encoding each luciferase having a different spectral characteristic of luminescence, so as to be detected distinguishably from a promotor region of other differentiation state detection marker gene, and
the image capturing step is a step of capturing each luminescence image corresponding to a spectral characteristic specific to each luciferase to obtain plural luminescence images.

4. The method according to any one of claims 1 to 3, **characterized in that** the image capturing step is a step of continuously capturing a plurality of luminescence images by a luminescence imaging system.

5. The method according to claim 4, further comprising a step of measuring the luminescence intensity as an index of the differentiation state in a specific cellular region of the luminescence images obtained in the image capturing step, and analyzing a temporal change of the luminescence intensity.

6. The method according to anyone of claims 1 to 5, further comprising a step of performing bright field observation and/or fluorescence observation for obtaining the information of the contour and/or site of the stem cell or the colony to which the stem cell belongs, and obtaining a bright field image and/or fluorescence image in the same region as the luminescence images.

7. The method according to any one of claims 1 to 6, wherein the stem cell is a stem cell into which two types of fusion genes are introduced,
one of the fusion genes is a fusion gene of a promoter region of an undifferentiation marker gene and a first type of a luciferase-coding gene, and the other is a fusion gene of a promoter region of a differentiation marker gene which is specifically expressed in a specific differentiation process and a second type of a luciferase-coding gene whose expression is detected distinguishably from the expression of the first type of the luciferase-coding gene.

8. A method for identifying the differentiation state of a stem cell, **characterized by** comprising a step of identifying the differentiation state of a stem cell, based on the luminescence image and/or luminescence intensity data and/or bright field image obtained by the method according to anyone of claims 1 to 6.

9. A method for collecting a stem cell showing a desired differentiation state, comprising
a step of identifying the differentiation state of a stem cell, based on the luminescence image and/or luminescence intensity data and/or bright field image obtained by a method according to any one of claims 1 to 6; and
a step of picking up a stem cell showing a desired differentiation state from a plurality of the stem cells, based on the identified differentiation state.

10. The method according to any one of claims 1 to 6, wherein the colony forms an embryoid body.

## Patentansprüche

1. Verfahren zum Überwachen des Differenzierungsstatus jeder Stammzelle in einer Stammzellenkolonie, umfassend:
einen Schritt (A-1) des Kultivierens von Stammzellen, in die ein Fusionsgen einer Promotorregion eines Differenzierungsstatusnachweismarkergens und eines luciferasecodierenden Gens transfiziert ist;
einen Schritt (A-2) des Kultivierens der Stammzellen unter einer differenzierungsinduzierenden Bedingung nach Schritt (A-1); und
einen Schritt (A-3) des Erfassens von Bildern einer Lumineszenz, die durch Expression des luciferasecodierenden Gens in den Stammzellen emittiert wird, über zumindest einen angegebenen Zeitraum der Schritte (A-1) bis (A-2), um Lumineszenzbilder zu erhalten, die die Expressionshöhe des luciferasecodierenden Gens jeder Stammzelle in der Kolonie indizieren,
wobei die Stammzelle eine Stammzelle ist, in die mehrere Typen von Fusionsgenen transfiziert sind,
jedes Fusionsgen eine Promotorregion eines unterschiedlichen Typs eines Differenzierungsstatusnachweismarkergens enthält,
jede Promotorregion des Differenzierungsstatusnachweismarkergens mit einem Gen fusioniert ist, das für jede Luciferase mit einem unterschiedlichen spektralen Lumineszenzcharakteristikum codiert, um von einer Promotorregion eines anderen Differenzierungsstatusnachweismarkergens unterscheidbar nachgewiesen zu werden, und
der Bilderfassungsschritt ein Schritt des Erfassens jedes Lumineszenzbilds ist, das einem spektralen Charakteristikum entspricht, das für jede Luciferase spezifisch ist, um mehrere Lumineszenzbilder zu erhalten.

2. Verfahren zum Überwachen des Differenzierungsstatus jeder Stammzelle in einer Stammzellenkolonie, umfassend:
einen Schritt (B-1) des Kultivierens von Stammzellen, in die ein Fusionsgen einer Promotorregion eines Differenzierungsstatusnachweismarkergens und eines luciferasecodierenden Gens eingebracht ist;
einen Schritt (B-2) des weiteren Subkultivierens der Stammzellen;
einen Schritt (B-3) des Kultivierens der Stammzellen unter einer differenzierungsinduzierenden Bedingung nach Schritt (B-2); und
einen Schritt (B-4) des Erfassens von Bildern einer Lumineszenz, die durch Expression des luciferasecodierenden Gens in den Stammzellen emittiert wird, über zumindest einen angegebenen Zeitraum der Schritte (B-1) bis (B-3), um Lumineszenzbilder zu erhalten, die die Expressionshöhe des luciferasecodierenden Gens jeder Stammzelle in der Kolonie indizieren,
wobei die Stammzelle eine Stammzelle ist, in die mehrere Typen von Fusionsgenen transfiziert sind,
jedes Fusionsgen eine Promotorregion eines unterschiedlichen Typs eines Differenzierungsstatusnachweismarkergens enthält,
jede Promotorregion des Differenzierungsstatusnachweismarkergens mit einem Gen fusioniert ist, das für jede Luciferase mit einem unterschiedlichen spektralen Lumineszenzcharakteristikum codiert, um von einer Promotorregion eines anderen Differenzierungsstatusnachweismarkergens unterscheidbar nachgewiesen zu werden, und
der Bilderfassungsschritt ein Schritt des Erfassens jedes Lumineszenzbilds ist, das einem spektralen Charakteristikum entspricht, das für jede Luciferase spezifisch ist, um mehrere Lumineszenzbilder zu erhalten.

3. Verfahren zum Überwachen des Differenzierungsstatus jeder Stammzelle in einer Stammzellenkolonie, umfassend:
einen Schritt (C-1) des Kultivierens von Stammzellen, in die ein Fusionsgen einer Promotorregion eines Differenzierungsstatusnachweismarkergens und eines luciferasecodierenden Gens eingebracht ist;
einen Schritt (C-2) des weiteren Subkultivierens der Stammzellen; und
einen Schritt (C-3) des Erfassens von Bildern einer Lumineszenz, die durch Expression des luciferasecodierenden Gens in den Stammzellen emittiert wird, über zumindest einen angegebenen Zeitraum der Schritte (C-1) bis (C-2), um Lumineszenzbilder zu erhalten, die die Expressionshöhe des luciferasecodierenden Gens jeder Stammzelle in der Kolonie indizieren,
wobei die Stammzelle eine Stammzelle ist, in die mehrere Typen von Fusionsgenen transfiziert sind,
jedes Fusionsgen eine Promotorregion eines unterschiedlichen Typs eines Differenzierungsstatusnachweismarkergens enthält,
jede Promotorregion des Differenzierungsstatusnachweismarkergens mit einem Gen fusioniert ist, das für jede Luciferase mit einem unterschiedlichen spektralen Lumineszenzcharakteristikum codiert, um von einer Promotorregion eines anderen Differenzierungsstatusnachweismarkergens unterscheidbar nachgewiesen zu werden, und
der Bilderfassungsschritt ein Schritt des Erfassens jedes Lumineszenzbilds ist, das einem spektralen Charakteristikum entspricht, das für jede Luciferase spezifisch ist, um mehrere Lumineszenzbilder zu erhalten.

4. Verfahren nach einem der Ansprüche 1 bis 3, **dadurch gekennzeichnet, dass** der Bilderfassungsschritt ein Schritt des kontinuierlichen Erfassens einer Mehrzahl von Lumineszenzbildern durch ein Lumineszenzbildgebungssystem ist.

5. Verfahren nach Anspruch 4, ferner umfassend einen Schritt des Messens der Lumineszenzintensität als Index für den Differenzierungsstatus in einer spezifischen Zellregion der Lumineszenzbilder, die im Bilderfassungsschritt erhalten wurden, und des Analysierens einer temporalen Änderung der Lumineszenzintensität.

6. Verfahren nach einem der Ansprüche 1 bis 5, ferner umfassend einen Schritt des Durchführens einer Hellfeldbeobachtung und/oder einer Fluoreszenzbeobachtung, um die Information zur Kontur und/oder Stelle der Stammzelle oder der Kolonie, zu der die Stammzelle gehört, zu erhalten und um ein Hellfeldbild und/oder ein Fluoreszenzbild in der gleichen Region wie die Lumineszenzbilder zu erhalten.

7. Verfahren nach einem der Ansprüche 1 bis 6, wobei die Stammzelle eine Stammzelle ist, in die zwei Typen von Fusionsgenen eingebracht sind,
eines der Fusionsgene ein Fusionsgen einer Promotorregion eines Undifferenzierungsmarkergens und eines ersten Typs eines luciferasecodierenden Gens ist, und
das andere ein Fusionsgen einer Promotorregion eines Differenzierungsmarkergens, das in einem spezifischen Differenzierungsprozess spezifisch exprimiert wird, und eines zweiten Typs eines luciferasecodierenden Gens ist, dessen Expression von der Expression des ersten Typs des luciferasecodierenden Gens unterscheidbar nachgewiesen wird.

8. Verfahren zum Identifizieren des Differenzierungsstatus einer Stammzelle, **dadurch gekennzeichnet, dass** es einen Schritt des Identifizierens des Differenzierungsstatus einer Stammzelle auf Basis des Lumineszenzbilds und/oder der Lumineszenzintensitätsdaten und/oder des Hellfeldbilds, wie mit dem Verfahren nach einem der Ansprüche 1 bis 6 erhalten, umfasst.

9. Verfahren zum Sammeln einer Stammzelle, die einen gewünschten Differenzierungsstatus zeigt, umfassend:
einen Schritt des Identifizierens des Differenzierungsstatus einer Stammzelle auf Basis des Lumineszenzbilds und/oder der Lumineszenzintensitätsdaten und/oder des Hellfeldbilds, wie mit einem Verfahren nach einem der Ansprüche 1 bis 6 erhalten; und
einen Schritt des Aufgreifens einer Stammzelle, die einen gewünschten Differenzierungsstatus zeigt, aus einer Mehrzahl der Stammzellen auf Basis des identifizierten Differenzierungsstatus.

10. Verfahren nach einem der Ansprüche 1 bis 6, wobei die Kolonie einen embryoiden Körper bildet.

## Revendications

1. Procédé pour surveiller l'état de différenciation de chaque cellule souche dans une colonie de cellules souches, comprenant :
une étape (A-1) consistant à cultiver des cellules couches dans lesquelles un gène de fusion d'une région de promoteur d'un gène marqueur de détection de l'état de différenciation et d'un gène codant pour la luciférase est transfecté ;
une étape (A-2) consistant à cultiver les cellules souches sous une condition d'induction de différenciation après l'étape (A-1) ; et
une étape (A-3) consistant à capturer des images de luminescence émise par l'expression du gène codant pour la luciférase dans les cellules souches, pendant au moins une période donnée des étapes (A-1) à (A-2), pour obtenir des images de luminescence indiquant le taux d'expression du gène codant pour la luciférase de chaque cellule souche dans la colonie,
la cellule souche étant une cellule souche dans laquelle plusieurs types de gènes de fusion sont transfectés,
chaque gène de fusion contenant une région de promoteur d'un type différent d'un gène marqueur de détection de l'état de différenciation,
chaque région de promoteur du gène marqueur de détection de l'état de différenciation étant fusionnée avec un gène codant pour chaque luciférase ayant une caractéristique spectrale de luminescence différente, de façon à être détectée de manière à pouvoir être distinguée d'une région de promoteur d'un autre gène marqueur de détection de l'état de différenciation, et
l'étape de capture d'image étant une étape consistant à capturer chaque image de luminescence correspondant à une caractéristique spectrale spécifique à chaque luciférase pour obtenir plusieurs images de luminescence.

2. Procédé pour surveiller l'état de différenciation de chaque cellule souche dans une colonie de cellules souches, comprenant :
une étape (B-1) consistant à cultiver des cellules souches dans lesquelles un gène de fusion d'une région de promoteur d'un gène marqueur de détection de l'état de différenciation et d'un gène codant pour la luciférase est introduit ;
une étape (B-2) consistant en outre à mettre en sous-culture les cellules souches ;
une étape (B-3) consistant à cultiver les cellules souches sous une condition d'induction de différenciation après l'étape (B-2) ; et
une étape (B-4) consistant à capturer des images de luminescence émise par l'expression du gène codant pour la luciférase dans les cellules souches, pendant au moins une période donnée des étapes (B-1) à (B-3), pour obtenir des images de luminescence indiquant le taux d'expression du gène codant pour la luciférase de chaque cellule souche dans la colonie,
la cellule souche étant une cellule souche dans laquelle plusieurs types de gènes de fusion sont transfectés,
chaque gène de fusion contenant une région de promoteur d'un type différent d'un gène marqueur de détection de l'état de différenciation,
chaque région de promoteur du gène marqueur de détection de l'état de différenciation étant fusionnée avec un gène codant pour chaque luciférase ayant une caractéristique spectrale de luminescence différente, de façon à être détectée de manière à pouvoir être distinguée d'une région de promoteur d'un autre gène marqueur de détection de l'état de différenciation, et
l'étape de capture d'image étant une étape consistant à capturer chaque image de luminescence correspondant à une caractéristique spectrale spécifique à chaque luciférase pour obtenir plusieurs images de luminescence.

3. Procédé pour surveiller l'état de différenciation de chaque cellule souche dans une colonie de cellules souches, comprenant :
une étape (C-1) consistant à cultiver des cellules souches dans lesquelles un gène de fusion d'une région de promoteur d'un gène marqueur de détection de l'état de différenciation et d'un gène codant pour la luciférase est introduit ;
une étape (C-2) consistant en outre à mettre en sous-culture les cellules souches ; et
une étape (C-3) consistant à capturer des images de luminescence émise par l'expression du gène codant pour la luciférase dans les cellules souches, pendant au moins une période donnée des étapes (C-1) à (C-2), pour obtenir des images de luminescence indiquant le taux d'expression du gène codant pour la luciférase de chaque cellule souche dans la colonie,
la cellule souche étant une cellule souche dans laquelle plusieurs types de gènes de fusion sont transfectés,
chaque gène de fusion contenant une région de promoteur d'un type différent d'un gène marqueur de détection de l'état de différenciation,
chaque région de promoteur du gène marqueur de détection de l'état de différenciation étant fusionnée avec un gène codant pour chaque luciférase ayant une caractéristique spectrale de luminescence différente, de façon à être détectée de manière à pouvoir être distinguée d'une région de promoteur d'un autre gène marqueur de détection de l'état de différenciation, et
l'étape de capture d'image étant une étape consistant à capturer chaque image de luminescence correspondant à une caractéristique spectrale spécifique à chaque luciférase pour obtenir plusieurs images de luminescence.

4. Procédé selon l'une quelconque des revendications 1 à 3, **caractérisé en ce que** l'étape de capture d'image est une étape consistant à capturer de manière continue une pluralité d'images de luminescence par un système d'imagerie de luminescence.

5. Procédé selon la revendication 4, comprenant en outre une étape consistant à mesurer l'intensité de luminescence en tant qu'indice de l'état de différenciation dans une région cellulaire spécifique des images de luminescence obtenues dans l'étape de capture d'image, et à analyser une modification temporelle de l'intensité de luminescence.

6. Procédé selon l'une quelconque des revendications 1 à 5, comprenant en outre une étape consistant à réaliser une observation sur fond clair et/ou une observation par fluorescence pour obtenir les informations sur le contour et/ou le site de la cellule souche ou de la colonie à laquelle la cellule souche appartient, et à obtenir une image sur fond clair et/ou une image en fluorescence dans la même région que les images de luminescence.

7. Procédé selon l'une quelconque des revendications 1 à 6, dans lequel la cellule souche est une cellule souche dans laquelle deux types de gènes de fusion sont introduits,
l'un des gènes de fusion est un gène de fusion d'une région de promoteur d'un gène marqueur de non-différenciation et d'un premier type d'un gène codant pour la luciférase, et l'autre est un gène de fusion d'une région de promoteur d'un gène marqueur de différenciation qui est exprimé de manière spécifique dans un processus de différenciation spécifique et d'un second type d'un gène codant pour la luciférase dont l'expression est détectée de manière à pouvoir être distinguée de l'expression du premier type du gène codant pour la luciférase.

8. Procédé pour identifier l'état de différenciation d'une cellule souche, **caractérisé en ce qu'**il comprend une étape consistant à identifier l'état de différenciation d'une cellule souche, sur la base de l'image de luminescence et/ou de données d'intensité de luminescence et/ou d'une image sur fond clair obtenue par le procédé selon l'une quelconque des revendications 1 à 6.

9. Procédé pour collecter une cellule souche présentant un état de différenciation souhaité, comprenant
une étape consistant à identifier l'état de différenciation d'une cellule souche, sur la base de l'image de luminescence et/ou de données d'intensité de luminescence et/ou d'une image sur fond clair obtenue par un procédé selon l'une quelconque des revendications 1 à 6 ; et
une étape consistant à prélever une cellule souche présentant un état de différenciation souhaité parmi une pluralité des cellules souches, sur la base de l'état de différenciation identifié.

10. Procédé selon l'une quelconque des revendications 1 à 6, dans lequel la colonie forme un corps embryoïde.
